# EUROPEAN PATENT APPLICATION

(11) **EP 2 743 042 A2**
(43) Date of publication of application: **18.06.2014**
(21) Application number: 12822094.4
(22) Date of filing: 13.08.2012
(51) Int. Cl.: B25J 19/00, B25J 17/00, A61B 17/062

(54) **APPARATUS FOR FIXING A JOINT STRUCTURE**

(30) Priority: 11.08.2011 KR 20110080182
(71) Applicant: Movasu, Inc., Seoul 110-450 (KR)
(72) Inventor: JEONG, Chang Wook, Seoul 110-450 (KR); KIM, Hyung Tae, Seoul 110-450 (KR); SIN, Chun Chol, Seoul 110-450 (KR)
(74) Representative: Zardi, Marco
(86) International application number: PCT/KR2012/006444
(87) International publication number: WO 2013/022325

(57) **Abstract**

The present invention relates to an apparatus for fixing a joint structure. According to an aspect of the present invention, the apparatus for fixing the joint structure includes: a positioning guide; at least three sliders; a lock member for locking each of the at least three sliders; and at least three links for connecting each of the at least three sliders to the positioning guide.

## Description

### FIELD OF THE INVENTION

The present invention relates to an apparatus for fixing a joint structure.

### BACKGROUND

Joint motion refers to the movement of at least one of two or more interconnected (mechanical) elements relative to the other ones. Such joint motion may be observed in various forms from human arms or legs to robotic arms of an industrial robot.

In some cases, instruments (particularly surgical instruments) capable of joint motion require to fix the joint motion state of the mechanical elements thereof. In order to fix the joint structure, devices or components configured with balls and sockets as disclosed in Korean Patent Application No. 2011-3192 of the applicant(s) (the contents of which are to be regarded as being incorporated herein by reference in its entirety) have been conventionally used, which mainly rely upon the surface friction between two mechanical elements. However, such devices or components have often been inadequate to achieve sufficiently firm and efficient fixation.

Herein, the inventor(s) thus present a novel apparatus for fixing a joint structure.

### SUMMARY OF THE INVENTION

One object of the present invention is to solve all the above problems in prior art.

Another object of the invention is to provide a novel apparatus for fixing a joint structure.

Yet another object of the invention is to provide a small-sized apparatus for fixing a joint structure.

Still another object of the invention is to provide an apparatus capable of effectively fixing a joint structure without relying upon surface friction.

According to one aspect of the invention to achieve the objects as described above, there is provided an apparatus for fixing a joint structure, comprising: a positioning guide; at least three sliders; lock members for locking each of the at least three sliders; and at least three links for connecting each of the at least three sliders to the positioning guide.

According to another aspect of the invention, there is provided a surgical instrument, comprising: an end effector; a joint unit for allowing the end effector to carry out joint motion; and an apparatus for fixing the joint motion state of the joint unit, wherein the fixing apparatus comprises a positioning guide; at least three sliders; lock members for locking each of the at least three sliders; and at least three links for connecting each of the at least three sliders to the positioning guide, and wherein the at least three sliders are capable of operating in a pitch or yaw direction relative to the positioning guide, together with a rotating element disposed in association with the positioning guide, and the joint unit and the rotating element are connected to each other by means of at least one wire.

In addition, there may be further provided other configurations according to the technical idea of the invention.

According to the invention, there is provided a novel apparatus for fixing a joint structure.

According to the invention, there is provided a small-sized apparatus for fixing a joint structure.

According to the invention, there is provided an apparatus capable of effectively fixing a joint structure without relying upon surface friction.

### BRIEF DESCRIPTION OF THE DRAWINGS

Fig. 1 is a conceptual diagram of an apparatus for fixing a joint structure according to one embodiment of the invention.
Fig. 2 is a conceptual diagram (cross-sectional view) of a rail for guiding the slide of a slider shown in Fig. 1.
Fig. 3 is another conceptual diagram of an apparatus for fixing a joint structure according to one embodiment of the invention.
Fig. 4 is a conceptual diagram of the principle of the joint structure fixing apparatus according to the invention.
Fig. 5 is a perspective view partially illustrating the interior of a joint structure fixing apparatus of a surgical instrument specifically implemented according to the invention.

### DETAILED DESCRIPTION OF THE PREFERRED EMBODIMENTS

In the following detailed description of the invention, references are made to the accompanying drawings that show, by way of illustration, specific embodiments in which the invention may be practiced. These embodiments are described in sufficient detail to enable those skilled in the art to practice the invention. It is to be understood that the various embodiments of the invention, although different from each other, are not necessarily mutually exclusive. For example, specific shapes, structures, or characteristics described herein may be implemented as modified from one embodiment to another without departing from the spirit and the scope of the invention. Furthermore, it shall be understood that the locations or arrangements of individual elements within each embodiment may be also modified without departing from the spirit and the scope of the invention. Therefore, the following detailed description is not to be taken in a limiting sense, and the scope of the invention is to be taken as encompassing the scope of the appended claims and all equivalents thereof. In the drawings, like reference numerals refer to the same or similar elements throughout the several views.

Hereinafter, preferred embodiments of the present invention will be described in detail with reference to the accompanying drawings to enable those skilled in the art to easily implement the invention.

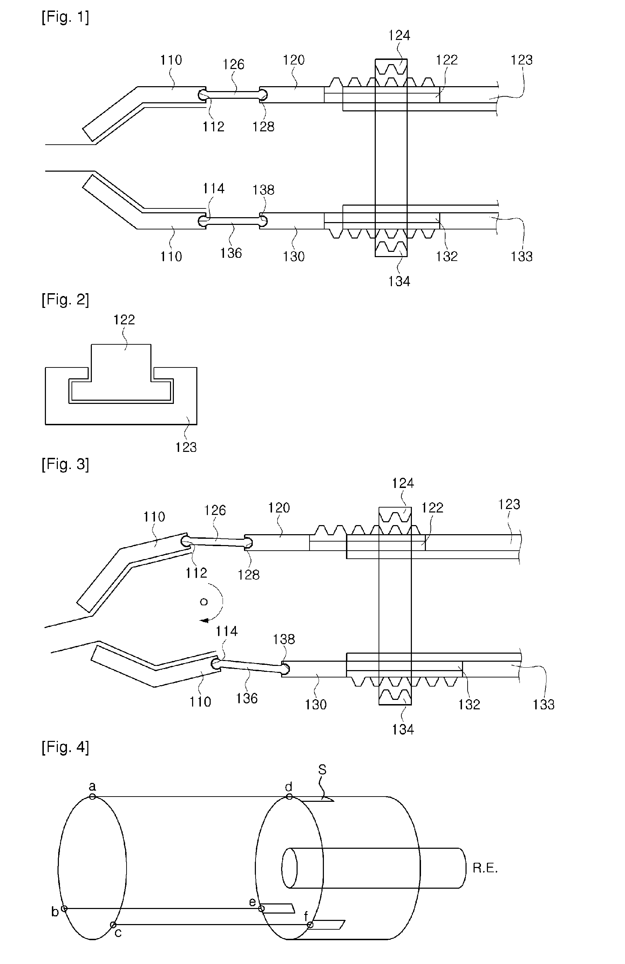

Fig. 1 is a conceptual diagram of an apparatus for fixing a joint structure according to one embodiment of the invention. Fig. 2 is a conceptual diagram (cross-sectional view) of a rail 123 for guiding the slide of a slider 120 shown in Fig. 1. In the following, reference will be made to Figs. 1 and 2.

The joint structure fixing apparatus according to the invention may comprise a positioning guide 110; at least three sliders 120, 130 (the illustration of a slider 140 being omitted here for convenience); lock members 122, 132 constituting at least a part of the sliders 120, 130 and lock members 124, 134 for engaging therewith to implement fixation; rails 123, 133 for guiding the slide of the sliders 120, 130; and links 126, 136 for connecting the positioning guide 110 and the sliders 120, 130. Here, both ends of the links 126, 136 may be received in connecting parts 112, 114 of the positioning guide 110 and connecting parts 128, 138 of the sliders 120, 130, respectively.

First, the positioning guide 110 may guide the position of a rotating element (not shown) therein (or adjacent thereto). (In connection with this, reference may be made to the elements 122 and 115 shown in the drawings of Korean Patent Application No.2011-3192.) The positioning guide 110 may be integrally formed, and the shape thereof may be an open-ended hemisphere, funnel or the like. Thus, the rotating element disposed in association with the positioning guide 110 may operate in a pitch or yaw direction according to the shape of the positioning guide 110. Further, the rotating element may cause joint motion by means of wires (not shown) connected thereto and extended through the open end of the positioning guide 110. The rotating element may also be fixed relative to the positioning guide 110.

The slider 120 will now be discussed. (Detailed description of the slider 130, which may have the substantially same configuration as the slider 120, will be omitted below.) The slider 120 may slide in only one direction along the rail 123, and may comprise the lock member 122 so that the position thereof may be fixed by the action of the lock member 122 and the corresponding external lock member 124. As shown in Fig. 1, the lock members 122, 124 may comprise convex and concave structures that may engage with each other to firm the above fixation. However, the shape or material of the lock members 122, 124 may be determined without limitation as long as the position of the slider 120 may be effectively fixed. Meanwhile, the rail 123 may be configured to allow the slider 120 to slide in the groove thereof as shown in Fig. 2. However, the configuration of the slider 120 or the rail 123 may be diversely changed as long as the slider 120 may slide in only one direction on the rail 123 without much wobble.

Further, both ends of the link 126 may be received in the connecting part 112 of the positioning guide 110 and the connecting part 128 of the slider 120 to connect the positioning guide 110 and the slider 120. Furthermore, the specified length of the link 126 may allow the rotating element disposed in association with the positioning guide 110 to operate in a pitch or yaw direction relative to the positioning guide 110 or to be fixed if necessary.

Both ends of the link 126 will be further discussed. As shown in Fig. 1, both ends of the link 126 may be in the shape of a sphere similar to a ball bearing. Further, the connecting part 112 of the positioning guide 110 and the connecting part 128 of the slider 120 may be in the shape to receive the corresponding ends of the link 126 so that the link 126 may move relatively freely around each of the ends.

Meanwhile, although the slider 120 or sliders 120, 130 have been mainly described above, it should be noted that the joint structure fixing apparatus according to the invention may preferably comprise three or more sliders and corresponding associated elements (preferably having configurations similar to each other) according to the principle of the invention as will be described below.

Fig. 3 is another conceptual diagram of an apparatus for fixing a joint structure according to one embodiment of the invention.

As shown in Fig. 3, the sliders 120, 130 and the associated elements thereof may operate together with the rotating element therebetween (e.g., around the rotation axis of the rotating element) in a pitch or yaw direction relative to the positioning guide 110. In this case, the positions of the links 126, 136 may be changed to allow the above operation as shown. The position changes may occur as the slider 120 slides in the left direction in the drawing along the rail 123 and the slider 130 slides in the right direction in the drawing along the rail 133. Thereafter, when the connecting parts 124, 134 operate to engage with the lock members 122, 132 according to a user's manipulation or the like, the sliders 120, 130 and the associated elements thereof may be fixed just as having operated together with the rotating element in a pitch or yaw direction relative to the positioning guide 110. (Naturally, in this case as well, the rotating element (or the torque transmission member therein) may at least operate freely in a surge or roll direction.) In this case, the fixation caused by the lock members 122, 124 or the like is sufficiently firm in contrast to that caused by a tightenable socket device (i.e., relying upon the surface friction between two elements) as disclosed in the above-mentioned Korean Patent Application 2011-3192.

Fig. 4 is a conceptual diagram of the principle of the joint structure fixing apparatus according to the invention.

In Fig. 4, points a through f abstractly represent where the links are connected to the positioning guide or the sliders, R.E. abstractly represents the rotating element, and S abstractly represents the slider. The positions of points a to c are fixed (i.e., the positioning guide is fixed) and those of points d to f are fixed relative to the rotating element (i.e., the sliders are fixed relative to the rotating element.) If the lengths of the links are specified, the rotating element may be fixed relative to the positioning guide even though it may operate together with the sliders in a pitch or yaw direction relative to the positioning guide when the sliders are not locked. That is, when the sliders are locked, the operation of the rotating element in a pitch or yaw direction relative to the positioning guide may be restricted.

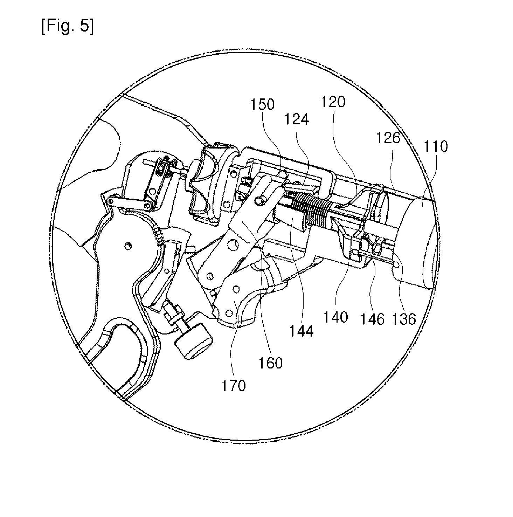

Fig. 5 is a perspective view partially illustrating the interior of a joint structure fixing apparatus of a surgical instrument specifically implemented according to the invention.

As shown in Fig. 5, the entire surgical instrument including the joint structure fixing apparatus may comprise a positioning guide 110; sliders 120, 140; lock members 124, 144; links 126, 136, 146; a fastening ring 150; a fastening ring press member 160; a presser 170; and a rotating element (not shown for being disposed inside the positioning guide 110). Although not all shown in Fig. 5, the entire surgical instrument may comprise a total of three sliders, each of which may be interlocked with a corresponding associated element such as a lock member.

First, when the fastening ring 150 is pressed by the fastening ring press member 160, it fastens the lock members 124, 144 or the like so that each of them may fix the slide state of the corresponding slider.

Further, the fastening ring press member 160 may be configured to rest in its initial position as being connected to a flat spring (not shown) and press the fastening ring 150 in the direction of the sliders 120, 140 upon a user's manipulation of pressing the presser 170. Meanwhile, the positions of the fastening ring press member 160 and the presser 170 may be restored by the action of the flat spring upon the user's release of the pressing force.

Further, the rotating element may allow an end effector (not shown) of the surgical instrument to carry out joint motion by means of wires that may be connected between the rotating element and a joint unit (not shown) adjacent to the end effector. In order to allow the joint motion, the rotating element may operate in a pitch or yaw direction relative to the positioning guide 110, which may be fixed relative to a patient's body. In addition, the joint motion state of the end effector caused thereby may be fixed upon the user's manipulation of pressing the presser 170 as described above.

Meanwhile, the elements similar to those described above in detail or those specifically disclosed in Korean Application Nos. 2011-3192, 2011-26243 and the like (the contents of which are to be regarded as being incorporated herein by reference in its entirety) will not be described herein.

Although the present invention has been described in terms of specific items such as detailed elements as well as the limited embodiments and the drawings, they are only provided to help general understanding of the invention, and the present invention is not limited to the above embodiments. It will be appreciated by a person of ordinary skill in the art that various modifications and changes may be made from the above description.

Therefore, the spirit of the present invention shall not be limited to the above-described embodiments, and the entire scope of the appended claims and their equivalents will fall within the scope and spirit of the invention.

## Claims

1. An apparatus for fixing a joint structure, comprising:
a positioning guide;
at least three sliders;
lock members for locking each of the at least three sliders; and
at least three links for connecting each of the at least three sliders to the positioning guide.

2. An apparatus for fixing a joint structure as claimed in Claim 1, wherein each of the at least three sliders is capable of sliding along a corresponding rail.

3. An apparatus for fixing a joint structure as claimed in Claim 2, wherein the lock members comprise convex and concave structures for locking the slide of each of the at least three sliders.

4. An apparatus for fixing a joint structure as claimed in Claim 1, wherein both ends of each of the at least three links are received in the positioning guide and the corresponding one of the at least three sliders.

5. An apparatus for fixing a joint structure as claimed in Claim 4, wherein each of the both ends is in the shape to allow the corresponding link to move around it.

6. An apparatus for fixing a joint structure as claimed in Claim 1, wherein the positioning guide is fixed, and
wherein the at least three sliders are capable of operating in a pitch or yaw direction relative to the positioning guide, together with a rotating element disposed in association with the positioning guide.

7. A surgical instrument, comprising:
an end effector;
a joint unit for allowing the end effector to carry out joint motion; and
an apparatus for fixing the joint motion state of the joint unit,
wherein the fixing apparatus comprises:
a positioning guide;
at least three sliders;
lock members for locking each of the at least three sliders; and
at least three links for connecting each of the at least three sliders to the positioning guide, and
wherein the at least three sliders are capable of operating in a pitch or yaw direction relative to the positioning guide, together with a rotating element disposed in association with the positioning guide, and the joint unit and the rotating element are connected to each other by means of at least one wire.

8. A surgical instrument as claimed in Claim 7, further comprising:
a fastening ring for actuating the lock members; and
a fastening ring press member for pressing and actuating the fastening ring.
